# EUROPEAN PATENT SPECIFICATION

(11) **EP 4 293 003 B1**
(45) Date of publication and mention of the grant of the patent: **26.06.2024**
(21) Application number: 23160451.3
(22) Date of filing: 07.03.2023
(51) Int. Cl.: C07C 1/24, C07C 7/10, C07C 7/13, C07C 11/04

(54) **PREPARATION OF ETHYLENE BY ETHANOL DEHYDRATION AND DEVICE THEREOF**
HERSTELLUNG VON ETHYLEN DURCH ETHANOLDEHYDRIERUNG UND VORRICHTUNG DAFÜR
PRÉPARATION D'ÉTHYLÈNE PAR DÉSHYDRATATION D'ÉTHANOL ET DISPOSITIF ASSOCIÉ

(30) Priority: 15.06.2022 CN 202210671939
(43) Date of publication of application: 20.12.2023
(73) Proprietor: Tianjin University, Tianjin 300350 (CN)
(72) Inventor: ZHANG, Minhua, JINNAN DISTRICT - TIANJIN, 300350 (CN); GONG, Hao, JINNAN DISTRICT - TIANJIN, 300350 (CN); DONG, He, JINNAN DISTRICT - TIANJIN, 300350 (CN); SHI, Feng, JINNAN DISTRICT - TIANJIN, 300350 (CN); YU, Yingzhe, JINNAN DISTRICT - TIANJIN, 300350 (CN)
(74) Representative: Cabinet Beau de Loménie

(56) References cited:
- CN-A- 101 244 970
- CN-A- 113 045 372
- CN-U- 207 871 848

## Description

### TECHNICAL FIELD

The present invention relates to a preparation of ethylene by ethanol dehydration and device thereof, in particular to a dehydrating and drying process and device for an ethylene molecular sieve.

### BACKGROUND ART

Ethylene is the most important organic chemical raw material, and its industrial scale, yield and technical level become important symbols for the development of the national chemical industry. Throughout the world, ethylene raw materials in industrialized countries are mainly light raw materials. Ethylene produced with naphtha as a cracking raw material accounts for about 50% of the total ethylene yield in the world; ethane is the second largest cracking raw material, and ethylene produced by cracking of the ethane accounts for about 28% of the total ethylene yield in the world; and the ethylene produced by cracking of the above two raw materials goes beyond 75% of the total yield, and the rest of ethylene is mainly prepared from liquefied petroleum gas (LPG), condensate oil, middle distillate and the like as raw materials.

The preparation of ethylene from ethanol is achieved by ethanol dehydration under the action of appropriate temperature and catalysts, and the preparation of ethylene by the catalytic dehydration of ethanol is the earliest technological method for the preparation of ethylene in industry. Unlike fossil raw materials for the preparation of ethylene, the raw material for the preparation of ethylene from ethanol is ethanol, which may be obtained by fermentation of biomass. Biomass is characterized by renewability, low pollution and wide distribution. Important biomass capable of supplying energy includes wood, wood wastes, crops, wastes generated during food processing, aquatic plants and the like. The process route of the preparation of ethylene from biomass ethanol also has the advantages of short construction period, relatively less investment, mild production conditions and the like; and in the preparation of ethylene by the production process, CO₂ emissions are reduced, and products have high purity and simple compositions, so that they are relatively easy to separate and purify.

The technological process of the preparation of ethylene by dehydrating ethanol usually includes two parts of an ethanol dehydration reaction section and an ethylene product purification section. Ethanol raw materials enter a dehydration reactor after being evaporatively preheated to generate crude ethylene, then the crude ethylene sequentially enters a washing tower, an alkaline washing tower, a drying tower, a de-light component tower, a de-heavy component tower and the like, so as to remove polar materials, CO₂, H₂O, light component byproducts, heavy component byproducts and the like, and finally ethylene products are obtained on a top of the de-heavy component tower.

Reaction products generated in the preparation of ethylene from ethanol contain a large amount of water, the crude ethylene also contains a large amount of water after being leached with desalted water in the alkaline washing tower, ethylene needs to be refined at low temperature, and if the water is brought into a low-temperature separation system, it will freeze at low temperature, leading to blockage of equipment and pipelines; and in addition to blockage caused by freezing of the water at low temperature, the water and hydrocarbons may generate white crystalline hydrates at high pressure and low temperature, and these hydrates will also be accumulated in the pipelines to cause blockage, so that it is necessary to dry ethylene before rectification.

A molecular sieve main body of a traditional molecular sieve drying device may be incapable of adsorbing water vapor after dehydrating and drying introduced gas materials for a period of time, at this moment, it is necessary for workers to replace the molecular sieve main body, however, the replacement operation is usually complicated, with time and labor wasted; it is necessary to stop drying to-be-dehydrated gas within the time for replacing the molecular sieve main body, in which reduces the working efficiency of the device; and it is necessary to manually monitor whether the molecular sieve main body needs to be replaced constantly, so that the automation degree is low.

CN201210248326.7 disclosed a regeneration method and equipment for a molecular sieve, which describes space gas replacement and thermal regeneration of a molecular sieve adsorption device achieving adsorption saturation with N₂ in detail. However, the method needs to perform material replacement before the termination of regeneration if being used for a drying tower of an ethylene device, leading to waste of a large quantity of materials and time in the process, and certain pollution will be caused due to N₂ emptying.

In CN201910089250.X, a crystal structure is reconstructed by blending and stirring an inactivated drying agent with raw materials for preparing a molecular sieve, however, this method is not suitable for a closed continuous dehydration molecular sieve.

CN113045372A discloses a process for the preparation of ethylene which comprises the dehydration of ethanol, cooling/separation in a quench compression system, alkaline washing and treatment with molecular sieves to remove CO₂ and water.

The present invention aims at providing a novel molecular sieve drying process for a device for preparing ethylene by ethanol dehydration. Internal circulation of ethylene is achieved by introducing part of ethylene, as desorbed gas, in an ethylene purification system in the device into a molecular sieve system for regeneration of the drying tower, thereby solving the problems of material waste and environmental pollution caused by the use of other desorbed gases.

### SUMMARY OF THE PRESENT INVENTION

The present invention relates to a preparation of ethylene by ethanol dehydration, in particular to a dehydrating and drying process and method for an ethylene molecular sieve. The present invention aims at providing a novel molecular sieve drying process for a device for preparing ethylene by ethanol dehydration.

A technical solution of the present invention is as follows:
A preparation of ethylene by ethanol dehydration, includes an ethanol dehydration reaction system, a quenching compression system, an alkaline washing system, a molecular sieve drying system, an ethylene purification system and a propylene refrigeration cycle system; and a technical solution adopted by the molecular sieve drying system includes the following steps:
(1) feeding crude ethylene and water from the alkaline washing system into the molecular sieve drying system, and after removing the water therefrom, feeding the obtained crude ethylene into the ethylene purification system; and
(2) feeding part of ethylene, acting as circulating ethylene, produced by the ethylene purification system into the molecular sieve drying system to serve as desorbed gas for regeneration of drying towers; and feeding the desorbed circulating ethylene back to the quenching compression system after cooling compression.

In the process for preparing the ethylene by ethanol dehydration, the molecular sieve drying system includes a circulating ethylene preheater, ethylene drying towers, ethylene drying tower protectors, a circulating ethylene cooler, a circulating ethylene compressor unit and a complete set of heating and conveying equipment; the ethylene drying towers and the protectors thereof need to be regenerated after adsorption saturation; the regeneration process consists of depressurizing, heating, cooling and pressurizing processes; and in the depressurizing process, gases exhausted out of the ethylene drying towers and the protectors thereof are fed into a quenching tower after being boosted by the circulating ethylene compressor unit. After pressure of the drying towers is decreased to a set value, the circulating ethylene from the circulating ethylene preheater is used for purging and heating from bottom to top, and the circulating ethylene is firstly heated to a set value by the circulating ethylene preheater to perform purging and heating on beds of the drying towers. Heating stops after temperature of the beds rises to a set value, and the circulating ethylene continues to perform purging and cooling on the beds of the drying towers; gases exhausted during heating and cooling of the beds are cooled by the circulating ethylene cooler and fed into the quenching tower after being boosted by the circulating ethylene compressor unit; and after the drying towers are cooled to certain temperature, firstly, the circulating ethylene is used for pressurizing the beds, after pressure of the beds reaches a set value, high-pressure dry ethylene exhausted out of the drying towers during adsorption operation continues to pressurize the beds into a balanced state, and the regenerated drying towers wait for entering a next adsorption/regeneration cycle.

Pressure of desorption operation of the drying towers is 0.06-2.00 MPa, and the temperature of desorption operation is 100°C-140°C.

The present invention provides a device for performing the preparation of ethylene by ethanol dehydration. In the molecular sieve drying system, gas phase outlets of gas-liquid separation tanks are respectively connected with tops of a first ethylene drying tower and a second ethylene drying tower through pipelines; a tower kettle of the first ethylene drying tower is respectively connected with tops of a first ethylene drying tower protector and a second ethylene drying tower protector; a tower kettle of the second ethylene drying tower is respectively connected with the tops of the first ethylene drying tower protector and the second ethylene drying tower protector; the tower kettle of the first ethylene drying tower, a tower kettle of the first ethylene drying tower protector, the tower kettle of the second ethylene drying tower and a tower kettle of the second ethylene drying tower protector are connected with an outlet in a heating side of the circulating ethylene preheater; the tops of the first ethylene drying tower and the second ethylene drying tower are connected with an inlet in a cooling side of the circulating ethylene cooler; an outlet in the cooling side of the circulating ethylene cooler is connected with an inlet of the circulating ethylene compressor unit; and the tower kettles of the first ethylene drying tower protector and the second ethylene drying tower protector are connected with an inlet in a cooling side of a circulating ethylene precooler.

The present invention has following advantages and beneficial effects:

As the novel crude ethylene dehydrating process and device, the preparation and device for preparing the ethylene by ethanol dehydration in the present invention have the advantages that part of the products of ethylene, acting as the circulating ethylene, in the ethylene purification system provide the desorbed gas for regeneration of the drying towers. By using the circulating ethylene as the desorbed gas, not only pollution caused by other desorbed gases (such as nitrogen) is avoided, but also the green production level is improved. The maximum adsorption capacity of the molecular sieve type 3A may reach 20%, the water content obtained after the circulating ethylene is desorbed is about 2% to 6%, and the water content of the dried crude ethylene may be below 1 ppm, which is far less than the standard water content of 5 ppm entering the ethylene purification system.

### BRIEF DESCRIPTION OF THE DRAWINGS

FIG. 1 is a flow diagram of an ethanol dehydration reaction system and a quenching compression system of a preparation of ethylene by ethanol dehydration according to the present invention.
FIG. 2 is a flow diagram of an alkaline washing system and a molecular sieve drying system of a preparation of ethylene by ethanol dehydration according to the present invention.
FIG. 3 is a flow diagram of an ethylene purification system and a propylene refrigeration cycle system of a preparation of ethylene by ethanol dehydration according to the present invention.

### DETAILED DESCRIPTION OF THE EMBODIMENTS

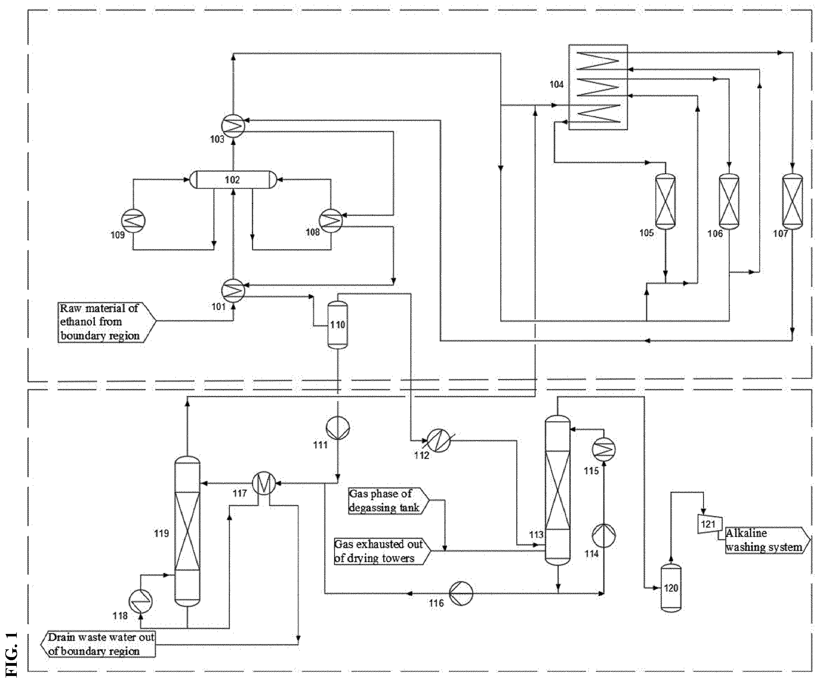

The present invention is further described in detail below with reference to FIGs. 1, 2 and 3 and specific embodiments. The following embodiments are merely descriptive and not restrictive, and do not limit the scope of protection of the present invention.

The present invention provides a preparation of ethylene by ethanol dehydration, including the following technical solution:
(1) Ethanol is fed into an ethanol dehydration reaction system for dehydration reaction to obtain reaction gas;
(2) the reaction gas is cooled in a quenching compression system and separated therefrom, and recovered steam returns to the ethanol dehydration reaction system;
(3) crude ethylene, carbon dioxide and water are fed into an alkaline washing system to remove the carbon dioxide component therefrom, so as to obtain the crude ethylene and the water;
(4) the crude ethylene and the water obtained from the alkaline washing system are fed into a molecular sieve drying system, and after the water therefrom is removed, the obtained crude ethylene is fed into an ethylene purification system;
(5) part of ethylene production, acting as circulating ethylene, produced by the ethylene purification system are fed into the molecular sieve drying system to supply desorbed gas for regeneration of drying towers;
(6) the crude ethylene is fed into the ethylene purification system to obtain ethylene products; and
(7) a propylene refrigeration cycle system supplies heat and cold to the ethylene purification system.

The present invention provides a novel production device for preparing ethylene from ethanol, including an ethanol preheater 101, an ethanol evaporation tank 102, an ethanol superheater 103, a heating furnace 104, a first reactor 105, a second reactor 106, a third reactor 107, a first ethanol evaporator 108, a second ethanol evaporator 109, a first gas-liquid separation tank 110, a separation tank discharge pump 111, a product cooler 112, a quenching tower 113, a quenching tower circulating pump 114, a quenching tower cooler 115, a quenching tower bottoms pump 116, a condensate preheater 117, an evaporation tower reboiler 118, an evaporation tower 119, a second gas-liquid separation tank 120, a first ethylene compressor 121, a first ethylene cooler 122, an alkaline washing tower 123, a crude ethylene cooler 124, an alkaline liquor circulating pump 125, a third gas-liquid separation tank 126, a circulating ethylene preheater 133, a first ethylene drying tower 127, a first ethylene drying tower protector 128, a second ethylene drying tower 129, a second ethylene drying tower protector 130, a circulating ethylene cooler 131, a circulating ethylene compressor unit 132, a degassing tank 134, a steam stripping tower 135, a steam stripping tower kettle water pump 136, a steam stripping tower kettle water cooler 137, an ethylene precooler 138, an ethylene evaporator 139, a fuel gas tank 140, a fuel gas heater 141, a circulating ethylene heater 142, a secondary propylene compressor gas supplementing tank 143, an ethylene chiller 144, a demethanizing tower 145, a demethanizing tower reboiler 146, a purification tower 147, a purification tower reboiler 148, a propylene collecting tank 149, a propylene condenser 150, a demethanizing tower cooler 151, a purification tower reflux tank 152, a purification tower top condenser 153, a primary propylene compressor gas supplementing tank 154, a primary propylene compressor 155, a secondary propylene compressor 156 and a complete set of heating and conveying equipment.

The equipment of the molecular sieve drying system is connected in such a manner that a gas phase outlet of the third gas-liquid separation tank 126 is respectively connected with tops of the first ethylene drying tower 127 and the second ethylene drying tower 129; a tower kettle of the first ethylene drying tower 127 is respectively connected with tops of the first ethylene drying tower protector 128 and the second ethylene drying tower protector 130; a tower kettle of the second ethylene drying tower 129 is respectively connected with the tops of the first ethylene drying tower protector 128 and the second ethylene drying tower protector 130; the tower kettle of the first ethylene drying tower 127, a tower kettle of the first ethylene drying tower protector 128, the tower kettle of the second ethylene drying tower 129 and a tower kettle of the second ethylene drying tower protector 130 are connected with an outlet in a heating side of the circulating ethylene preheater 133; the tops of the first ethylene drying tower 127 and the second ethylene drying tower 129 are connected with an inlet in a cooling side of the circulating ethylene cooler 131; an outlet in the cooling side of the circulating ethylene cooler 131 is connected with an inlet of the circulating ethylene compressor unit 132; and the tower kettles of the first ethylene drying tower protector 128 and the second ethylene drying tower protector 130 are connected with an inlet in a cooling side of the circulating ethylene precooler 138.

In the above technical solution, a specific implementation of step (1) is as follows: a raw material of ethanol enters the ethanol evaporation tank 102 for evaporation after being preheated by the ethanol preheater 101, and reaction gas generated by the reaction of heated ethanol steam through the heating furnace and the reactors enters the first gas-liquid separation tank 110 after its heat is recovered. A separated liquid phase is fed into the evaporation tower 119, and a gas phase is fed into the quenching tower 113.

In the above technical solution, a specific implementation of step (2) is as follows: the reaction gas from the ethanol dehydration system is fed into the quenching tower 113 to be further cooled after being cooled, streams obtained on a top of the quenching tower 113 are fed into the second gas-liquid separation tank 120 for gas-liquid separation, and crude ethylene is obtained and fed into the alkaline washing system. A liquid phase in a tower kettle of the quenching tower 113 and the reaction gas are heat exchanged to obtain condensate, and the obtained condensate is fed into the evaporation tower 119 after being preheated, recovered steam obtained on the top of the evaporation tower 119 is fed back to the ethanol dehydration reaction system to serve as diluted steam, and residual waste liquor obtained in a tower kettle of the evaporation tower 119 is fed to the outside of a boundary region for waste liquor treatment.

In the above technical solution, a specific implementation of step (3) is as follows: the crude ethylene from the quenching compression system is fed into a bottom of the alkaline washing tower 123 after being cooled, and desalted water is fed into a top of the alkaline washing tower 123 for leaching, so as to remove a carbon dioxide component from the crude ethylene. Waste alkaline liquor and waste water in a tower kettle are fed into the degassing tank 134 after converging. Ethylene, water and the like obtained on the top of the alkaline washing tower 123 are fed into the third gas-liquid separation tank 126 after being cooled. A separated gas phase is fed into the molecular sieve drying system, and a liquid phase is fed into the degassing tank 134. A gas phase exhausted out of the degassing tank 134 returns to the quenching tower 113, and a liquid phase enters the steam stripping tower 135. A gas phase separated out of the steam stripping tower 135 is fed to the outside of the boundary region for waste gas treatment, and tower bottoms are fed to the outside of the boundary region for sewage treatment after being cooled.

In the above technical solution, a specific implementation of step (4) is as follows: the crude ethylene and the water fed from the alkaline washing system enter the first ethylene drying tower 127 and the second ethylene drying tower 129 and the protectors thereof, and dry crude ethylene obtained after dehydrating and drying is fed into the ethylene purification system for further treatment.

In the above technical solution, a specific implementation of step (5) is as follows: first ethylene drying tower 127 and the second ethylene drying tower 129 and the protectors thereof need to be regenerated after adsorption saturation. The regeneration process consists of depressurizing, heating, cooling and pressurizing processes. In the depressurizing process, gases exhausted out of first ethylene drying tower 127 or the second ethylene drying tower 129 and the protector thereof are fed into the quenching tower 113 after being boosted by the circulating ethylene compressor unit 132. After pressure of the drying towers is decreased to 0.15-0.18 MPa, the circulating ethylene from the circulating ethylene preheater 133 is used for purging and heating from bottom to top, and the circulating ethylene is firstly heated to 100°C-140°C by the circulating ethylene preheater 133 before performing purging and heating on beds of the drying towers. Heating stops after temperature of the beds rises to a set value, and the circulating ethylene continues to perform purging and cooling on the beds of the drying towers. Gases exhausted during heating and cooling of the beds are cooled by the circulating ethylene cooler 131 and fed into the quenching tower 113 after being boosted by the circulating ethylene compressor unit 132. After temperature of an absorber is cooled to 100°C-140°C, firstly, the circulating ethylene is used for pressurizing the beds to 1.7-2.0 MPa, and then high-pressure dry ethylene exhausted out of the drying towers during adsorption operation continues to pressurize the beds into a balanced state. The regenerated drying towers wait for entering a next adsorption/regeneration cycle.

In the above technical solution, a specific implementation of step (6) is as follows: the crude ethylene from the molecular sieve drying system is fed into the demethanizing tower 145 after being cooled, one part of light component impurities obtained on a top of the demethanizing tower 145 reflux, and the other part thereof is fed into the fuel gas tank 140; and crude ethylene obtained on a tower kettle of the demethanizing tower 145 is fed into the purification tower 147, and materials in the tower kettle are fed into the fuel gas tank 140. Overhead gas enters the purification tower reflux tank 152 after being condensed, one part of ethylene serves as reflux liquid, the other part thereof is adiabatically flashed to obtain low-temperature ethylene, and the low-temperature ethylene is fed into the molecular sieve drying system. The rest of products of ethylene in the purification tower reflux tank 152 are heated to set temperature to obtain ethylene products, and the ethylene products are fed to the outside of the boundary region.

In the above technical solution, a specific implementation of step (7) is as follows: the propylene refrigeration cycle system supplies heat and cold to the ethylene purification system, and a liquid phase of propylene obtained after heat exchange is fed into the propylene collecting tank 149. The liquid phase of propylene in the propylene collecting tank 149 is fed into the primary propylene compressor gas supplementing tank 154 for gas-liquid separation after being cooled, the liquid phase of propylene is temporarily stored in the tank, and a gas phase enters the primary propylene compressor 156 after being compressed.

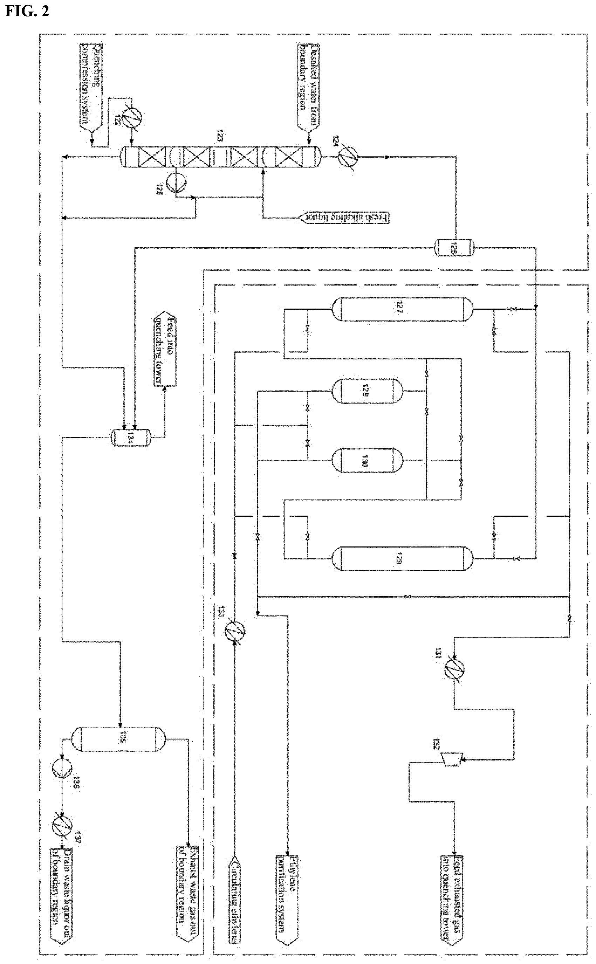

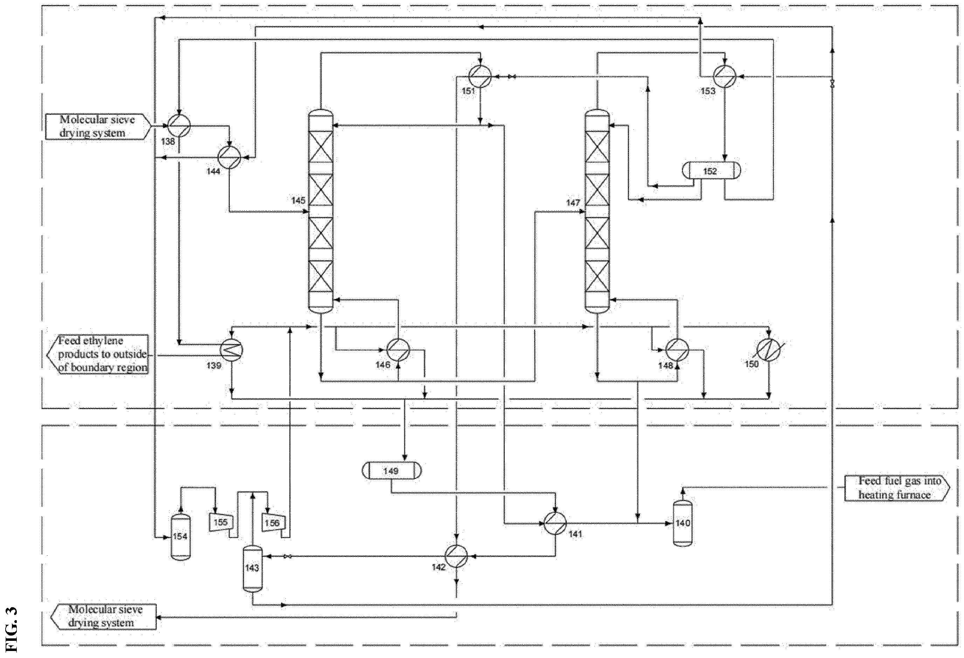

FIG. 1 shows a flow diagram of the ethanol dehydration reaction system and the quenching compression system of the process for preparing ethylene by ethanol dehydration of the present invention, FIG. 2 shows a flow diagram of the alkaline washing system and the molecular sieve drying system, and FIG. 3 shows a flow diagram of the ethylene purification system and the propylene refrigeration cycle system.

Operating pressure of the first reactor is 0.75-1.25 MPa, inlet temperature is 420°C-520°C, and outlet temperature is 300°C-430°C; operating pressure of the second reactor is 0.55-0.95 MPa, inlet temperature is 420°C-520°C, and outlet temperature is 300°C-430°C; operating pressure of the third reactor is 0.40-0.70 MPa, inlet temperature is 420°C-520°C, and outlet temperature is 300°C-430°C; operating pressure of the quenching tower is 0.25-0.55 MPa, top temperature is 30°C-60°C, and tower kettle temperature is 70°C-90°C; operating pressure of the evaporation tower is 1.20-1.45 MPa, top temperature is 180°C-225°C, and tower kettle temperature is 190°C-245°C; operating pressure of the alkaline washing tower is 1.50-2.30 MPa, top temperature is 20°C-46°C, and tower kettle temperature is 30°C-55°C; pressure of desorption operation of the drying towers is 0.06-2.00 MPa, and the temperature of desorption operation is 100°C-140°C; operating pressure of the steam stripping tower is 0.22-0.28 MPa, and operating temperature is 109°C-139°C. Operating pressure of the demethanizing tower is 1.55-2.35 MPa, top temperature ranges from -83°C to -56°C, and tower kettle temperature ranges from -43°C to -26°C; and operating pressure of the purification tower is 1.55-2.35 MPa, top temperature ranges from -43°C to -20°C, and tower kettle temperature ranges from -29°C to -9°C.

The specific implementation process of the method of the present application is described with the specific embodiments below.

### Embodiment 1:

A raw material of ethanol enters the ethanol evaporation tank 102 for evaporation after being preheated by the ethanol preheater 101, and reaction gas generated by the reaction of heated ethanol steam through the heating furnace and the reactors enters the first gas-liquid separation tank 110 after its heat is recovered. A separated liquid phase is fed into the evaporation tower 119, and a gas phase is fed into the quenching tower 113.

The reaction gas from the ethanol dehydration system is fed into the quenching tower 113 to be further cooled after being cooled, streams obtained on the top of the quenching tower 113 are fed into the second gas-liquid separation tank 120 for gas-liquid separation, and crude ethylene and the like are obtained and fed into the alkaline washing system; and a liquid phase in the tower kettle of the quenching tower 113 and the reaction gas are heat exchanged to obtain condensate, and the obtained condensate is fed into the evaporation tower 119 after being preheated, recovered steam obtained on the top is fed back to the ethanol dehydration reaction system to serve as diluted steam, and residual waste liquor obtained on the tower kettle of the evaporation tower 119 is fed to the outside of the boundary region for waste liquid treatment.

The crude ethylene from the quenching compression system is fed into the bottom of the alkaline washing tower 123 after being cooled, desalted water is fed into the top of the alkaline washing tower 123 for leaching, so as to remove a carbon dioxide component from the crude ethylene, and waste alkaline liquor and waste water in the kettle are fed into the degassing tank 134 after converging. Ethylene, water and the like obtained on the top of the alkaline washing tower 123 are fed into the third gas-liquid separation tank 126 after being cooled. A separated gas phase is fed into the molecular sieve drying system, and a liquid phase is fed into the degassing tank 134. A gas phase exhausted out of the degassing tank 134 returns to the quenching tower 113, and a liquid phase enters the steam stripping tower 135. A gas phase separated out of the steam stripping tower 135 is fed to outside of the boundary region for waste gas treatment, and tower bottoms are fed to the outside of the boundary region for sewage treatment after being cooled.

The crude ethylene fed from the alkaline washing system enters the first ethylene drying tower 127 and the second ethylene drying tower 129 and the protectors thereof for dehydrating and drying, and the crude ethylene obtained after dehydrating and drying is fed into the ethylene purification system. The water content of the dried crude ethylene is 0.9 ppm. The temperature of desorption operation of the drying towers is 100°C, and the pressure of desorption operation is 0.06 MPa.

The first ethylene drying tower 127 and the second ethylene drying tower 129 and the protectors thereof need to be regenerated with circulating ethylene as desorbed gas after adsorption saturation. The regeneration process consists of depressurizing, heating, cooling and pressurizing processes. In the depressurizing process, gases exhausted out of the first ethylene drying tower 127 or the second ethylene drying tower 129 and the protector thereof are fed into the quenching tower 113 after being boosted by the circulating ethylene compressor unit 132. After pressure of the drying towers is decreased to 0.15 MPa, the circulating ethylene from the circulating ethylene preheater 133 is used for purging and heating from bottom to top, and the circulating ethylene is firstly heated to 100°C by the circulating ethylene preheater 133 to perform purging and heating on the beds of the drying towers. Heating stops after temperature of the beds rises to a set value, and the circulating ethylene continues to perform purging and cooling on the beds of the drying towers. Gases exhausted during heating and cooling of the beds are cooled by the circulating ethylene cooler 131 and fed into the quenching tower 113 after being boosted by the circulating ethylene compressor unit 132. After temperature of an absorber is cooled to 100°C, firstly, the circulating ethylene is used for pressurizing the beds, and after pressure of the beds reaches 1.7 MPa, high-pressure dry ethylene exhausted out of the drying towers during adsorption operation continues to pressurize the beds into a balanced state. The regenerated drying towers wait for entering a next adsorption/regeneration cycle. The water content of a desorbed molecular sieve is 2%.

The crude ethylene from the molecular sieve drying system is fed into the demethanizing tower 145 after being cooled, one part of light component impurities obtained on the top of the demethanizing tower 145 reflux, and the other part thereof is fed into the fuel gas tank 140. Crude ethylene obtained in the tower kettle of the demethanizing tower 145 is fed into the purification tower 147, and materials in the tower kettle are fed into the fuel gas tank 140. Overhead gas enters the purification tower reflux tank 152 after being condensed, one part of ethylene serves as reflux liquid, the other part thereof is adiabatically flashed to obtain low-temperature ethylene, and the low-temperature ethylene is fed into the molecular sieve drying system. The rest of products of ethylene in the purification tower reflux tank 152 are heated to set temperature to obtain ethylene products, and the ethylene products are fed to the outside of the boundary region.

The propylene refrigeration cycle system supplies heat and cold to the ethylene purification system, and a liquid phase of propylene obtained after heat exchange is fed into the propylene collecting tank 149; and the liquid phase of propylene in the propylene collecting tank 149 is fed into the primary propylene compressor gas supplementing tank 154 for gas-liquid separation after being cooled, the liquid phase of propylene is temporarily stored in the tank, and a gas phase enters the primary propylene compressor 156 after being compressed.

A feed quantity of the raw material of ethylene in the embodiment is 4,200 kg/hr, ethylene products with a mass fraction of 99.91% are finally obtained, a conversion rate is 99.6%, 1.55 tons of steam is consumed for producing one ton of ethylene, and a regeneration period of a catalyst is 18 months.

### Embodiment 2:

A raw material of ethanol enters the ethanol evaporation tank 102 for evaporation after being preheated by the ethanol preheater 101, and reaction gas generated by the reaction of heated ethanol steam through the heating furnace and the reactors enters the first gas-liquid separation tank 110 after its heat is recovered. A separated liquid phase is fed into the evaporation tower 119, and a gas phase is fed into the quenching tower 113.

The reaction gas from the ethanol dehydration system is fed into the quenching tower 113 to be further cooled after being cooled, streams obtained on the top of the quenching tower 113 are fed into the second gas-liquid separation tank 120 for gas-liquid separation, and crude ethylene and the like are obtained and fed into the alkaline washing system; and a liquid phase in the tower kettle of the quenching tower 113 and the reaction gas are heat exchanged to obtain condensate, and the obtained condensate is fed into the evaporation tower 119 after being preheated, recovered steam obtained on the top of the evaporation tower 119 is fed back to the ethanol dehydration reaction system to serve as diluted steam, and residual waste liquor obtained in the tower kettle of the evaporation tower 119 is fed to the outside of the boundary region for waste liquid treatment.

The crude ethylene from the quenching compression system is fed into the bottom of the alkaline washing tower 123 after being cooled, desalted water is fed into the top for leaching, so as to remove a carbon dioxide component from the crude ethylene, and waste alkaline liquor and waste water in the tower kettle are fed into the degassing tank 134 after converging. Ethylene, water and the like obtained on the top of the alkaline washing tower 123 are fed into the third gas-liquid separation tank 126 after being cooled. A separated gas phase is fed into the molecular sieve drying system, and a liquid phase is fed into the degassing tank 134. A gas phase exhausted out of the degassing tank 134 returns to the quenching tower 113, and a liquid phase enters the steam stripping tower 135. A gas phase separated out of the steam stripping tower 135 is fed to outside of the boundary region for waste gas treatment, and tower bottoms are fed to the outside of the boundary region for sewage treatment after being cooled.

The crude ethylene fed from the alkaline washing system enters the first ethylene drying tower 127 and the second ethylene drying tower 129 and the protectors thereof for dehydrating and drying, and the crude ethylene obtained after dehydrating and drying is fed into the ethylene purification system. The water content of the dried crude ethylene is 0.85 ppm. The temperature of desorption operation of the drying towers is 150°C, and the pressure of desorption operation is 2.0 MPa.

The first ethylene drying tower 127 and the second ethylene drying tower 129 and the protectors thereof need to be regenerated with circulating ethylene as desorbed gas after adsorption saturation. The regeneration process consists of depressurizing, heating, cooling and pressurizing processes. In the depressurizing process, gases exhausted out of the first ethylene drying tower 127 or the second ethylene drying tower 129 and the protector thereof are fed into the quenching tower 113 after being boosted by the circulating ethylene compressor unit 132. After pressure of the drying towers is decreased to 0.18 MPa, the circulating ethylene from the circulating ethylene preheater 133 is used for purging and heating from bottom to top, and the circulating ethylene is firstly heated to 140°C by the circulating ethylene preheater 133 to perform purging and heating on the beds of the drying towers. Heating stops after temperature of the beds rises to a set value, and the circulating ethylene continues to perform purging and cooling on the beds of the drying towers. Gases exhausted during heating and cooling of the beds are cooled by the circulating ethylene cooler 131 and fed into the quenching tower 113 after being boosted by the circulating ethylene compressor unit 132. After temperature of an absorber is cooled to 140°C, firstly, the circulating ethylene is used for pressurizing the beds, and after pressure of the beds reaches 1.88 MPa, high-pressure dry ethylene exhausted out of the drying towers during adsorption operation continues to pressurize the beds into a balanced state. The regenerated drying towers wait for entering a next adsorption/regeneration cycle. The water content of a desorbed molecular sieve is 4%.

The crude ethylene from the molecular sieve drying system is fed into the demethanizing tower 145 after being cooled, one part of light component impurities obtained on the top of the demethanizing tower 145 reflux, and the other part thereof is fed into the fuel gas tank 140. Crude ethylene obtained in the tower kettle of the demethanizing tower 145 is fed into the purification tower 147, and materials in the tower kettle are fed into the fuel gas tank 140. Overhead gas enters the purification tower reflux tank 152 after being condensed, one part of ethylene serves as reflux liquid, the other part thereof is adiabatically flashed to obtain low-temperature ethylene, and the low-temperature ethylene is fed into the molecular sieve drying system. The rest of products of ethylene in the purification tower reflux tank 152 are heated to set temperature to obtain ethylene products, and the ethylene products are fed to the outside of the boundary region.

The propylene refrigeration cycle system supplies heat and cold to the ethylene purification system, and a liquid phase of propylene obtained after heat exchange is fed into the propylene collecting tank 149. The liquid phase of propylene in the propylene collecting tank 149 is fed into the primary propylene compressor gas supplementing tank 154 for gas-liquid separation after being cooled, the liquid phase of propylene is temporarily stored in the tank, and a gas phase enters the primary propylene compressor 156 after being compressed.

A feed quantity of the raw material of ethylene in the embodiment is 4,500 kg/hr, ethylene products with a mass fraction of 99.91% are finally obtained, a conversion rate is 99.6%, and 1.56 tons of steam is consumed for producing one ton of ethylene.

### Embodiment 3:

A raw material of ethanol enters the ethanol evaporation tank 102 for evaporation after being preheated by the ethanol preheater 101, and reaction gas generated by the reaction of heated ethanol steam through the heating furnace and the reactors enters the first gas-liquid separation tank 110 after its heat is recovered. A separated liquid phase is fed into the evaporation tower 119, and a gas phase is fed into the quenching tower 113.

The reaction gas from the ethanol dehydration system is fed into the quenching tower 113 to be further cooled after being cooled, streams obtained on the top of the quenching tower 113 are fed into the second gas-liquid separation tank 120 for gas-liquid separation, and obtained crude ethylene is fed into the alkaline washing system. A liquid phase in the tower kettle of the quenching tower 113 and the reaction gas are heat exchanged to obtain condensate, and the obtained condensate is fed into the evaporation tower 119 after being preheated, recovered steam obtained on the top of the evaporation tower 119 is fed back to the ethanol dehydration reaction system to serve as diluted steam, and residual waste liquor obtained in the tower kettle of the evaporation tower 119 is fed to the outside of the boundary region for waste liquid treatment.

The crude ethylene from the quenching compression system is fed into the bottom of the alkaline washing tower 123 after being cooled, desalted water is fed into the top for leaching, so as to remove a carbon dioxide component from the crude ethylene, and waste alkaline liquor and waste water in the tower kettle are fed into the degassing tank 134 after converging. Ethylene, water and the like obtained on the top of the alkaline washing tower 123 are fed into the third gas-liquid separation tank 126 after being cooled. A separated gas phase is fed into the molecular sieve drying system, and a liquid phase is fed into the degassing tank 134. A gas phase exhausted out of the degassing tank 134 returns to the quenching tower 113, and a liquid phase enters the steam stripping tower 135. A gas phase separated out of the steam stripping tower 135 is fed to the outside of the boundary region for waste gas treatment, and tower bottoms are fed to the outside of the boundary region for sewage treatment after being cooled.

The crude ethylene fed from the alkaline washing system enters the first ethylene drying tower 127 and the second ethylene drying tower 129 and the protectors thereof for dehydrating and drying, and the crude ethylene obtained after dehydrating and drying is fed into the ethylene purification system. The water content of the dried crude ethylene is 0.9 ppm. The temperature of desorption operation of the drying towers is 130°C, and the pressure of desorption operation is 1.00 MPa.

The first ethylene drying tower 127 and the second ethylene drying tower 129 and the protectors thereof need to be regenerated with circulating ethylene as desorbed gas after adsorption saturation. The regeneration process consists of depressurizing, heating, cooling and pressurizing processes. In the depressurizing process, gases exhausted out of the first ethylene drying tower 127 or the second ethylene drying tower 129 and the protector thereof are fed into the quenching tower 113 after being boosted by the circulating ethylene compressor unit 132. After pressure of the drying towers is decreased to 0.16 MPa, the circulating ethylene from the circulating ethylene preheater 133 is used for purging and heating from bottom to top, and the circulating ethylene is firstly heated to 130°C by the circulating ethylene preheater 133 to perform purging and heating on the beds of the drying towers. Heating stops after temperature of the beds rises to a set value, and the circulating ethylene continues to perform purging and cooling on the beds of the drying towers. Gases exhausted during heating and cooling of the beds are cooled by the circulating ethylene cooler 131 and fed into the quenching tower 113 after being boosted by the circulating ethylene compressor unit 132. After temperature of an absorber is cooled to 130°C, firstly, the circulating ethylene is used for pressurizing the beds, and after pressure of the beds reaches 1.9 MPa, high-pressure dry ethylene exhausted out of the drying towers during adsorption operation continues to pressurize the beds into a balanced state. The regenerated drying towers wait for entering a next adsorption/regeneration cycle. The water content of a desorbed molecular sieve is 6%.

The crude ethylene from the molecular sieve drying system is fed into the demethanizing tower 145 after being cooled, one part of light component impurities obtained on the top of the demethanizing tower 145 reflux, and the other part thereof is fed into the fuel gas tank 140; and crude ethylene obtained in the tower kettle of the demethanizing tower 145 is fed into the purification tower 147, and materials in the tower kettle are fed into the fuel gas tank 140. Overhead gas enters the purification tower reflux tank 152 after being condensed, one part of ethylene serves as reflux liquid, the other part thereof is adiabatically flashed to obtain low-temperature ethylene, and the low-temperature ethylene is fed into the molecular sieve drying system. The rest of products of ethylene in the purification tower reflux tank 152 are heated to set temperature to obtain ethylene products, and the ethylene products are fed to the outside of the boundary region.

The propylene refrigeration cycle system supplies heat and cold to the ethylene purification system, and a liquid phase of propylene obtained after heat exchange is fed into the propylene collecting tank 149. The liquid phase of propylene in the propylene collecting tank 149 is fed into the primary propylene compressor gas supplementing tank 154 for gas-liquid separation after being cooled, the liquid phase of propylene is temporarily stored in the tank, and a gas phase enters the primary propylene compressor 156 after being compressed.

A feed quantity of the raw material of ethylene in the embodiment is 4,800 kg/hr, ethylene products with a mass fraction of 99.91% are finally obtained, a conversion rate is 99.6%, and 1.57 tons of steam is consumed for producing one ton of ethylene produced.

The present invention has the technical beneficial effects that by adopting the circulating ethylene as the desorbed gas, regeneration operation of the drying towers is achieved by means of internal circulation of ethylene, so as to improve the green production level.

Equipment unspecified in the present invention is conventional equipment, and it can be achieved by adopting methods and equipment well known to those skilled in the art. Although the present invention has been described with reference to the specific implementation solutions and drawings, the present invention is not expected to be limited to the specific form here. On the contrary, the scope of the present invention is only limited by the appended claims. In addition, although independent features may be included in different claims, these features may be advantageously combined, and the inclusion in different claims does not mean that the combination of features is not feasible and/or advantageous. References to "first", "second," etc., do not exclude plurals.

## Claims

1. A preparation of ethylene by ethanol dehydration, comprising an ethanol dehydration reaction system, a quenching compression system, an alkaline washing system, a molecular sieve drying system, an ethylene purification system and a propylene refrigeration cycle system; wherein a technical solution adopted by the molecular sieve drying system comprises the following steps:
(1) feeding crude ethylene and water from the alkaline washing system into the molecular sieve drying system, and after removing the water therefrom, feeding the obtained crude ethylene into the ethylene purification system; and
(2) feeding part of ethylene, acting as circulating ethylene, produced by the ethylene purification system into the molecular sieve drying system to serve as desorbed gas for regeneration of drying towers; and feeding the desorbed circulating ethylene back to the quenching compression system after cooling compression.

2. The process for preparing the ethylene by ethanol dehydration according to claim 1, **characterized in that** the molecular sieve drying system comprises a circulating ethylene preheater, ethylene drying towers, ethylene drying tower protectors, a circulating ethylene cooler, a circulating ethylene compressor unit and a complete set of heating and conveying equipment; the ethylene drying towers and the protectors thereof need to be regenerated after adsorption saturation, and the regeneration process consists of depressurizing, heating, cooling and pressurizing processes; in the depressurizing process, gases exhausted by the ethylene drying towers and the protectors thereof are fed into a quenching tower after being boosted by the circulating ethylene compressor unit; after pressure of the drying towers is decreased to a set value, the circulating ethylene from the circulating ethylene preheater is used for purging and heating from bottom to top, and the circulating ethylene is firstly heated to a set value by the circulating ethylene preheater to perform purging and heating on beds of the drying towers; heating stops after temperature of the beds rises to a set value, and the circulating ethylene continues to perform purging and cooling on the beds of the drying towers; gases exhausted during heating and cooling of the beds are cooled by the circulating ethylene cooler and fed into the quenching tower after being boosted by the circulating ethylene compressor unit; and after the drying towers are cooled to certain temperature, firstly, the circulating ethylene is used for pressurizing the beds, after pressure of the beds reaches a set value, high-pressure dry ethylene exhausted out of the drying towers during adsorption operation continues to pressurize the beds into a balanced state, and the regenerated drying towers wait for entering a next adsorption/regeneration cycle.

3. The process for preparing the ethylene by ethanol dehydration according to claim 1 or 2, **characterized in that** pressure of desorption operation of the drying towers is 0.06-2.00 MPa, and the temperature of desorption operation is 100°C-140°C.

4. A device for performing the preparation of the ethylene by ethanol dehydration according to claim 1, 2 or 3, **characterized in that** in devices of the molecular sieve drying system, gas phase outlets of gas-liquid separation tanks are respectively connected with tops of a first ethylene drying tower and a second ethylene drying tower through pipelines; a tower kettle of the first ethylene drying tower is respectively connected with tops of a first ethylene drying tower protector and a second ethylene drying tower protector; a tower kettle of the second ethylene drying tower is respectively connected with the tops of the first ethylene drying tower protector and the second ethylene drying tower protector; the tower kettle of the first ethylene drying tower, a tower kettle of the first ethylene drying tower protector, the tower kettle of the second ethylene drying tower and a tower kettle of the second ethylene drying tower protector are connected with an outlet in a heating side of the circulating ethylene preheater; the tops of the first ethylene drying tower and the second ethylene drying tower are connected with an inlet in a cooling side of the circulating ethylene cooler; an outlet in the cooling side of the circulating ethylene cooler is connected with an inlet of the circulating ethylene compressor unit; and the tower kettles of the first ethylene drying tower protector and the second ethylene drying tower protector are connected with an inlet in a cooling side of a circulating ethylene precooler.

## Patentansprüche

1. Herstellung von Ethylen durch Ethanoldehydrierung, umfassend ein Ethanoldehydrierungsreaktionssystem, ein Abschreckverdichtungssystem, ein Alkaliwaschsystem, ein Molekularsiebtrocknungssystem, ein Ethylenreinigungssystem und ein Propylenkältezyklussystem, wobei eine technische Lösung, die durch das Molekularsiebtrocknungssystem übernommen wird, die folgenden Schritte umfasst:
(1) Zuführen von Rohethylen und Wasser von dem Alkaliwaschsystem in das Molekularsiebtrocknungssystem und nach dem Entfernen des Wassers daraus Zuführen des erhaltenen Rohethylens in das Ethylenreinigungssystem, und
(2) Zuführen eines Teils von Ethylen, das als zirkulierendes Ethylen agiert, produziert durch das Ethylenreinigungssystem, in das Molekularsiebtrocknungssystem, um als desorbiertes Gas für Regeneration von Trocknungstürmen zu dienen, und Zuführen des desorbierten zirkulierenden Ethylens zurück zu dem Abschreckverdichtungssystem nach Kühlverdichtung.

2. Verfahren zur Herstellung des Ethylens durch Ethanoldehydrierung nach Anspruch 1, **dadurch gekennzeichnet, dass** das Molekularsiebtrocknungssystem einen Vorheizer für zirkulierendes Ethylen, Ethylentrocknungstürme, Ethylentrocknungsturmprotektoren, einen Kühler für zirkulierendes Ethylen, eine Verdichtereinheit für zirkulierendes Ethylen und einen vollständigen Satz von Erwärmungs- und Förderausrüstung umfasst, wobei die Ethylentrocknungstürme und die Protektoren davon nach Adsorptionssättigung regeneriert werden müssen und das Regenerationsverfahren aus Druckverringerungs-, Erwärmungs-, Kühl- und Unterdrucksetzungsverfahren besteht, wobei in dem Druckverringerungsverfahren Gase, die durch die Ethylentrocknungstürme und die Protektoren davon erschöpft werden, in einen Abschreckturm zugeführt werden, nachdem sie durch die Verdichtereinheit für zirkulierendes Ethylen verstärkt werden, nachdem der Druck der Trocknungstürme auf einen festgelegten Wert verringert wurde, das zirkulierende Ethylen von dem Vorheizer für zirkulierendes Ethylen zum Spülen und Erwärmen von unten nach oben verwendet wird und das zirkulierende Ethylen zuerst auf einen festgelegten Wert durch den Vorheizer für zirkulierendes Ethylen erwärmt wird, um das Spülen und Erwärmen an Betten der Trocknungstürme durchzuführen, wobei das Erwärmen stoppt, nachdem die Temperatur der Betten auf einen festgelegten Wert ansteigt, und das zirkulierende Ethylen weiter das Spülen und Kühlen an den Betten der Trocknungstürme durchführt, Gase, die während des Erwärmens und Kühlens der Betten erschöpft werden, durch den Kühler für zirkulierendes Ethylen gekühlt und in den Abschreckturm zugeführt werden, nachdem sie durch die Verdichtereinheit für zirkulierendes Ethylen verstärkt werden, und nachdem die Trocknungstürme auf eine bestimmte Temperatur gekühlt wurden, zuerst das zirkulierende Ethylen zum Unterdrucksetzen der Betten verwendet wird, nachdem der Druck der Betten einen festgelegten Wert erreicht, trockenes Hochdruckethylen, das aus den Trocknungstürmen während eines Adsorptionsbetriebes erschöpft wird, weiter die Betten in einen ausgeglichenen Zustand unter Druck setzt, und die regenerierten Trocknungstürme darauf warten, in einen nächsten Adsorptions-/Regenerationszyklus einzutreten.

3. Verfahren zur Herstellung des Ethylens durch Ethanoldehydrierung nach Anspruch 1 oder 2, **dadurch gekennzeichnet, dass** der Druck des Desorptionsbetriebes der Trocknungstürme 0,06-2,00 MPa beträgt und die Temperatur des Desorptionsbetriebes 100 °-140°C beträgt.

4. Vorrichtung zum Durchführen der Herstellung des Ethylens durch Ethanoldehydrierung nach Anspruch 1, 2 oder 3, **dadurch gekennzeichnet, dass** bei Vorrichtungen des Molekularsiebtrocknungssystems Gasphasenauslässe von Gas-Flüssigkeit-Trenntanks jeweils mit Oberseiten eines ersten Ethylentrocknungsturms und eines zweiten Ethylentrocknungsturms durch Rohrleitungen verbunden sind, ein Turmkessel des ersten Ethylentrocknungsturms jeweils mit Oberseiten eines ersten Ethylentrocknungsturmprotektors und eines zweiten Ethylentrocknungsturmprotektors verbunden ist, ein Turmkessel des zweiten Ethylentrocknungsturms jeweils mit den Oberseiten des ersten Ethylentrocknungsturmprotektors und des zweiten Ethylentrocknungsturmprotektors verbunden ist, der Turmkessel des ersten Ethylentrocknungsturms, ein Turmkessel des ersten Ethylentrocknungsturmprotektors, der Turmkessel des zweiten Ethylentrocknungsturms und ein Turmkessel des zweiten Ethylentrocknungsturmprotektors mit einem Auslass in einer Erwärmungsseite des Vorheizers für zirkulierendes Ethylen verbunden sind, die Oberseiten des ersten Ethylentrocknungsturms und des zweiten Ethylentrocknungsturms mit einem Einlass in einer Kühlseite des Kühlers für zirkulierendes Ethylen verbunden sind, ein Auslass in der Kühlseite des Kühlers für zirkulierendes Ethylen mit einem Einlass der Verdichtereinheit für zirkulierendes Ethylen verbunden ist, und die Turmkessel des ersten Ethylentrocknungsturmprotektors und des zweiten Ethylentrocknungsturmprotektors mit einem Einlass in einer Kühlseite eines Vorkühlers für zirkulierendes Ethylen verbunden sind.

## Revendications

1. Préparation d'éthylène par déshydratation d'éthanol, comprenant un système de réaction de déshydratation d'éthanol, un système de compression avec désactivation, un système de lavage alcalin, un système de séchage sur tamis moléculaire, un système de purification d'éthylène et un système de cycle de réfrigération au propylène ; dans laquelle une solution technique adoptée par le système de séchage sur tamis moléculaire comprend les étapes suivantes :
(1) introduction d'éthylène brut et d'eau provenant du système de lavage alcalin dans le système de séchage sur tamis moléculaire et, après que l'eau en a été éliminée, introduction de l'éthylène brut obtenu dans le système de purification d'éthylène ; et
(2) introduction d'une partie de l'éthylène, agissant comme de l'éthylène en circulation, produit par le système de purification d'éthylène, dans le système de séchage sur tamis moléculaire afin qu'il serve de gaz désorbé pour la régénération des tours de séchage ; et réintroduction de l'éthylène en circulation désorbé dans le système de compression avec désactivation après la compression avec refroidissement.

2. Procédé pour préparer de l'éthylène par déshydratation d'éthanol selon la revendication 1, **caractérisé en ce que** le système de séchage sur tamis moléculaire comprend un préchauffeur d'éthylène en circulation, des tours de séchage d'éthylène, des protecteurs de tours de séchage d'éthylène, un refroidisseur d'éthylène en circulation, une unité de compresseur d'éthylène en circulation et un ensemble complet d'équipement de chauffage et de convoyage ; les tours de séchage d'éthylène et leurs protecteurs doivent être régénérés après saturation par adsorption, et le procédé de régénération consiste en des traitements de dépressurisation, chauffage, refroidissement et pressurisation ; dans le traitement de dépressurisation, les gaz sortant des tours de séchage d'éthylène et de leurs protecteurs sont introduits dans une tour de désactivation après avoir été renforcés par l'unité de compresseur d'éthylène en circulation ; après que la pression des tours de séchage a diminué à une valeur de consigne, l'éthylène en circulation provenant du préchauffeur d'éthylène en circulation est utilisé à des fins de purge et de chauffage de bas en haut, et l'éthylène en circulation est d'abord chauffé à une valeur de consigne par le préchauffeur d'éthylène en circulation pour réaliser une purge et un chauffage sur les lits des tours de séchage ; le chauffage s'arrête après que la température des lits a atteint une valeur de consigne, et l'éthylène en circulation continue de réaliser une purge et un refroidissement sur les lits des tours de séchage ; les gaz sortant durant le chauffage et le refroidissement des lits sont refroidis par le refroidisseur d'éthylène en circulation et introduits dans la tour de désactivation après avoir été renforcés par l'unité de compresseur d'éthylène en circulation ; et après que les tours de séchage ont été refroidies à une température de consigne, tout d'abord, l'éthylène en circulation est utilisé pour pressuriser les lits, après que la pression des lits a atteint une valeur de consigne, de l'éthylène sec sous haute pression sortant des tours de séchage durant l'opération d'adsorption continue de pressuriser les lits dans un état équilibré, et les tours de séchage régénérées attendent pour entrer dans un cycle suivant d'adsorption/régénération.

3. Procédé pour préparer de l'éthylène par déshydratation d'éthanol selon la revendication 1 ou 2, **caractérisé en ce que** la pression de l'opération de désorption des tours de séchage est de 0,06 à 2,00 MPa, et la température de l'opération de désorption est de 100 °C à 140 °C.

4. Dispositif pour réaliser la préparation d'éthylène par déshydratation d'éthanol selon la revendication 1, 2 ou 3, **caractérisé en ce que**, dans des dispositifs du système de séchage sur tamis moléculaire, les sorties de phase gazeuse de réservoirs de séparation gaz-liquide sont respectivement connectées aux sommets d'une première tour de séchage d'éthylène et d'une deuxième tour de séchage d'éthylène par l'intermédiaire de conduites ; une bouilloire de tour de la première tour de séchage d'éthylène est respectivement connectée aux sommets du premier protecteur de tour de séchage d'éthylène et au deuxième protecteur de tour de séchage d'éthylène ; une bouilloire de tour de la deuxième tour de séchage d'éthylène est respectivement connectée aux sommets du premier protecteur de tour de séchage d'éthylène et au deuxième protecteur de tour de séchage d'éthylène ; la bouilloire de tour de la première tour de séchage d'éthylène, une bouilloire de tour du premier protecteur de tour de séchage d'éthylène, la bouilloire de tour de la deuxième tour de séchage d'éthylène et une bouilloire de tour du deuxième protecteur de tour de séchage d'éthylène sont connectées à une sortie du côté chauffage du préchauffeur d'éthylène en circulation ; les sommets de la première tour de séchage d'éthylène et de la deuxième tour de séchage d'éthylène sont connectés à une entrée du côté refroidissement du refroidisseur d'éthylène en circulation ; une sortie du côté refroidissement du refroidisseur d'éthylène en circulation est connectée à une entrée de l'unité de compresseur d'éthylène en circulation ; et les bouilloires de tour du premier protecteur de tour de séchage d'éthylène et du deuxième protecteur de tour de séchage d'éthylène sont connectées à une entrée du côté refroidissement d'un pré-refroidisseur d'éthylène en circulation.
